# EUROPEAN PATENT SPECIFICATION

(11) **EP 1 143 808 B1**
(45) Date of publication and mention of the grant of the patent: **23.09.2009**
(21) Application number: 99941769.4
(22) Date of filing: 27.08.1999
(51) Int. Cl.: A23L 1/00

(54) **METHOD OF MAKING INGESTIBLE COMPOSITIONS COMPRISING ANTIBACTERIAL AGENTS**
VERFAHREN ZUR HERSTELLUNG VON ESSBAREN ZUSAMMENSTELLUNGEN MIT ANTIBAKTERIELLEN WIRKSTOFFEN
PROCEDE DE FABRICATION DE COMPOSITIONS POUVANT ETRE INGEREES, COMPRENANT DES AGENTS ANTIBACTERIENS

(30) Priority: 28.08.1998 GB 9818913; 10.12.1998 GB 9827243
(43) Date of publication of application: 17.10.2001
(73) Proprietor: UNIVERSITY OF BATH, Claverton Down, Bath, BA2 7AY (GB)
(72) Inventor: BLAKE, David, Russell, Bath BA1 2ET (GB); STEVENS, Clifford Robert, Bradford on Avon, Wiltshire BA15 1TL (GB); EISENTHAL, Robert, Bath BA1 4LR (GB); HARRISON, Roger, Bradford on Avon, Wiltshire BA15 1PH (GB); MILLAR, Timothy Mark, Southdown, Bath BA2 1LT (GB); EDWARDS, Rachel, Bradford on Avon, Wiltshire BA15 1HP (GB)
(74) Representative: Humphreys, Ceris Anne
(86) International application number: PCT/GB1999/002845
(87) International publication number: WO 2000/011965

(56) References cited:
- EP-A- 0 290 410
- EP-A- 0 477 143
- EP-A- 0 518 445
- WO-A-93/23080
- US-A- 5 310 541
- US-A- 5 645 834
- MILLAR T.M. ET AL: "Xanthine oxidase catalyses the reduction of nitrates and nitrite to nitric oxide under hypoxic conditions" FEBS LETTERS,May 1998 (1998-05), pages 225-228, XP002133526 cited in the application
- SOUCI; FACHMANN; KRAUT: 'Food Composition and Nutrition Tables', 1989, WISSENTSCHAFTLICHE VERLAGSGESELLSCHAFT, STUTTGART
- CERBULIS J.; FARRELL H.M.: 'Xanthine Oxidase Activity in Dairy Products' JOURNAL OF DAIRY SCIENCE vol. 60, no. 2, 1977, PHILADELPHIA, pages 170 - 176
- GREENBANK G.R.; PALLANSCH M.J.: 'Inactivation and Reactivation of Xanthine Oxidase in Dairy Products' J DAIRY SCIENCE vol. 45, 18 April 1962, WASHINGTON DC, pages 958 - 961

## Description

The present invention relates to compositions comprising xanthine oxidoreductase (XOR). In particular, but not exclusively, the invention relates to the use of a composition comprising XOR as a feed for humans or animals, and finds special application in relation to formula feed for babies.

The enzyme xanthine oxidoreductase (XOR) is a complex molybdoflavoprotein, the action of which has been studied for many years.

XOR is a major protein component of the membrane surrounding fat droplets in whole milk. Consequently, cows' milk is a rich and convenient source of the enzyme, as has been known for many years. XOR has also been characterised from rat, chicken and turkey livers. More recently, human milk has been found to contain XOR, which has now been purified from that source. Initial research shows that, while the human milk XOR enzyme has similar physicochemical properties to the bovine milk enzyme, the human enzyme shows differences in its catalytic activity.

The purpose of XOR in milk has never been fully ascertained. One suggestion has been that it provides a source of molybdenum and iron, metals potentially useful for the developing neonate.

There have also been suggestions that XOR has a role in the production of bactericidal agents. As discussed further below, XOR can catalyse the production of superoxide and hydrogen peroxide, which are known bactericidal agents. As indicated below, however, in the low oxygen concentrations found in the gut, it is thought unlikely that bactericidal levels of superoxide or hydrogen peroxide will be attained.

The role of XOR in the development of gout has been the subject of extensive study. XOR is involved in the catabolism of purines to uric acid, catalysing the oxidation of hypoxanthine to xanthine and xanthine to uric acid. Compositions have been developed which block the action of XOR and which are useful in the prevention and treatment of gout.

XOR exists in two inter-convertible forms, xanthine dehydrogenase (XDH, EC 1.1.1.204) and xanthine oxidase (XO, EC 1.1.3.22). XDH, which is believed to predominate *in vivo*, preferentially reduces NAD⁺, whereas XO does not reduce NAD⁺, preferring molecular oxygen. Where reference is made herein to xanthine oxidoreductase, it should be understood that that term refers to both xanthine dehydrogenase (XDH) and xanthine oxidase (XO), where appropriate. It will be appreciated that references to XOR further include references to analogs of xanthine oxidoreductase that have xanthine oxidoreductase activity. Such analogs may include but are not limited to, for example, xanthine oxidoreductase which has been modified chemically or otherwise, analogs having fragments of xanthine oxidoreductase derived from naturally-occurring enzyme, and analogs having polypeptides obtained by replication of the enzyme or a portion thereof using any suitable biotechnological method, provided in each case that the catalytic activity of the endogenous xanthine oxidoreductase is retained at least to an appreciable extent.

XOR can also effect reduction of molecular oxygen. That reaction generates the reactive oxygen species superoxide anion and hydrogen peroxide.

The superoxide radicals thus generated have been implicated in relation to chronic inflammatory intestinal diseases such as Crohn's disease and colitis ulcerosa. US 5,484,605 describes the administration of oxypurinol to the intestine to inhibit xanthine oxidase, thus preventing the formation of superoxide radicals which are believed to act as inflammatory mediators.

It has been reported, Millar, T.M. et al, FEBS Letters 427 (1998) 225-228, that, under hypoxic conditions and in the presence of NADH, XOR is capable of catalysing the reduction of glyceryl trinitrate (GTN), as well as inorganic nitrate and nitrite, to nitric oxide (NO).

US 5,645,834 describes a colostrum based supplement for cattle that contains many health enhancing factors and US 5,310,541 discloses a antimicrobial raw hide animal chew that contains an antimicrobial enzyme system. EP 0518445 discloses the use of the antimicrobial lacto peroxidase system in food stuff preservation.

Nitric oxide (NO) is widely recognised as mediating the relaxation of smooth muscle in vasodilation and as initiating many other important biological functions, including inhibition of platelet aggregation and adhesion. Its generally accepted physiological source is NO synthase, a complex enzyme which is totally dependent on oxygen for its activity and consequently ineffective in a hypoxic environment, where the vasodilatory properties of NO might be seen to be advantageous.

Babies who are not breast-fed are fed what is referred to herein as formula feed. Such formula feed has a composition which commonly includes sources of protein, fat and carbohydrate as well as minerals and are generally formulated to be nutritionally complete. Many formula feeds are based on cow's milk while some others are soy based. The term "formula feed" used herein should be understood to cover both formulations based on milk products as well as those based on soya or other products and which may or may not be nutritionally complete. The formula feed commonly takes the form of a dried powder which is reconstituted before being fed to the baby. Alternatively, the formula feed may be in liquid form either as a concentrated liquid requiring dilution or as a ready-to-feed formulation.

Enteral feeding may be prescribed where a patient is unable to eat normally, or has severe malabsorption or malnourishment. Enteral feed may take the form of tube and sip feeds. The enteral feed may be nutritionally complete and generally comprises protein, carbohydrate and fat as well as vitamins and minerals. The term "enteral feed" used herein should be understood to cover both formulations based on milk products as well as those based on soya or other products and which may or may not be nutritionally complete. The enteral feed may be in liquid form or may be in the form of a powder which is reconstituted before use. In many cases the enteral feed formulation is based on the composition of the neonatal formula feed.

The present invention provides a method of making a formula feed for a human baby, the method comprising manufacturing a powder feed formulation, the powder feed formulation so obtained being substantially free of active XOR and further comprising adding to that powder feed formulation a composition comprising active XOR.

The term "feed" is used herein to include both enteral feeds and formula feeds.

We have found that the active enzyme xanthine oxidoreductase (XOR) is absent from formula feeds and from enteral feeds. It is thought that that is because XOR is either absent from the feed or it is inactivated as a result of the treatment process used in the production of feeds. Where reference is made herein to "active" XOR and "active" enzyme, it is to be understood that reference is made to XOR and enzyme which has not, for example, been inactivated or broken down in such a way.

Up to now, it would have been thought that the addition of active XOR to feeds would be unnecessary and undesirable. The only roles of XOR previously known, as indicated above, were as a general "housekeeping" enzyme in the catabolism of purines, which may also have a pathological role in the development of gout and of chronic inflammatory intestinal diseases. The only other beneficial role of XOR was thought to be as a source of molybdenum and iron. It was thought (correctly) that those minerals could still be obtained from inactivated XOR, which might be present in feed.

We have now found that the addition of active XOR to feed is potentially beneficial in killing pathogenic intestinal bacteria. The active XOR may thus be regarded as a "natural antibiotic", that is, a substance of natural origin which is capable of destroying or inhibiting the growth of at least some strains of pathogenic micro-organism. It is believed that feed containing active XOR will be beneficial in the mitigation of intestinal infection and necrotising enterocolitis in the neonate fed with formula feed. Sick adults that are enterally fed are also at risk of the same complications as children fed formula feed.

Studies have shown that babies who are fed with formula feed are about twenty times more likely to suffer from gastrointestinal (GI) infection than babies who are breast fed. The reason for that discrepancy has not been known.

In the presence of oxygen, as stated above, XOR can generate superoxide and hydrogen peroxide. However, the amount of oxygen available in the gut is generally very low and bactericidal levels of superoxides or hydrogen peroxide are unlikely to be attained.

As discussed above, it has been found that the catalysis by XOR of the reduction of glyceryl trinitrate (GTN), as well as inorganic nitrate and nitrite, to nitric oxide (NO) occurs under hypoxic conditions. It is such hypoxic conditions which are present in the gut.

We have also found that the optimum pH for the production of NO under the hypoxic conditions is about pH 5.5. It is known that the pH of the neonatal stomach normally ranges between pH 3.5 and 6. Thus, we have found that the neonatal stomach presents an environment in which the production of NO in the presence of XOR is at a peak. Furthermore, it is at pH levels above about 4 at which pathogenic bacteria are more active than at lower pH of about 2 normally found in the adult stomach. Thus it is believed that the neonatal stomach, having a relatively high pH, is at particular risk from pathogenic bacteria. Furthermore, it is found that, for example in post-operative adults, the pH of the stomach rises to above 4. It has also been noted that post-operative adults have a relatively high risk of GI infection.

Thus, surprisingly, we have found under the conditions of the neonatal gut, XOR can catalyse the production not only of superoxide, but of NO. Superoxide and NO rapidly interact to generate peroxynitrite, a much more potent bactericidal species than superoxide, NO or hydrogen peroxide. While superoxide has some bactericidal properties and in some situations NO has also been found to kill or damage bacteria, it is the interaction of superoxide and NO to form peroxynitrite and other products which is believed to give superior bactericidal action. Peroxynitrite (and, it is thought, other products of the interaction of superoxide and NO) are particularly potent bactericidal species.

The concentration of nitrite present in the neonatal gut is normally low, and it might therefore be thought that the potential for XOR-catalysed generation of NO would be limited. It is believed, however, that the known affinity of XOR for acidic polysaccharides such as those occurring in bacterial capsules causes XOR to become more concentrated in the immediate vicinity of bacteria. In anaerobic environments, bacteria commonly are found to excrete nitrite and thus the association of XOR with the bacteria may have the result that the XOR will be located in a localised region of elevated nitrite concentration.

As indicated above, the use of XOR finds particular application in relation to the feeding of neonates.

Preferably, the amount of the composition comprising active XOR added is such that the concentration of the XOR in the formula feed, when reconstituted, exceeds the normal physiological concentration of XOR. Advantageously, the formula feed, when reconstituted, includes from 50 to 500µg/ml of XOR. Preferably the formulation includes from 50 to 150µg/ml which is comparable to the level of XOR found in natural breast milk. In some cases, it may be desirable to increase or reduce the amount of active XOR in the feed, for example, as a function of the baby's age and/or the incidence of GI infection.

We have found that a particularly advantageous source of active XOR is buttermilk, in particular lyophilised buttermilk.

In some cases it is thought that the addition of XOR in purified or other form will be desirable, for example where there is a risk of allergy to buttermilk.

The formula feed including the active XOR obtained in accordance with the invention may be in the form of a powder or may be in liquid form ready for feeding to the patient.

As indicated above, it is believed that for the formula feed formulations, any active XOR which was naturally present in those formulations is destroyed or deactivated during the manufacture of the formulation, possibly as a result of heat treatment. In a particularly preferred embodiment of the invention, one portion of the formula is prepared in the standard way, for example including heat treatment step(s). A composition comprising active XOR is subsequently added to the prepared portion. That addition of the XOR may be carried out by a manufacturer, or may be carried out, for example, immediately prior to use of the formulation. For example, in the case of a powdered feed which is made up with water prior to use, a powdered composition comprising the active XOR may be contained in a separate container from that of the rest of the formulation, for example in a sachet. Alternatively, the portion including the active XOR may be in tablet form or may be contained in a capsule. The rest of the feed may be made up in the normal way, for example by the addition of hot water and shaking, and the XOR composition added once the rest of the feed has been prepared. That is of particular importance in the case in which the normal method of making up of the formulation might deactivate the XOR, for example as a result of the addition of boiling water. Where the making up of the formulation would not damage the activity of the XOR, or where the formulation is sold ready-prepared, the XOR composition and the rest of the formulation may be provided together as a mixture.

Thus the method of the invention provides for manufacture of a combination product for use in the preparation of a formula feed, in which the product comprises two separate portions, the first portion including active XOR and the second portion being in powder form and comprising substantially no active XOR.

As indicated above, the second portion may have been treated by heat treatment, for example UHT treatment, in the normal way, thus deactivating the XOR.

In a particularly preferred embodiment of the invention, the active XOR is added in the form of buttermilk. The first portion may be pasteurised. As indicated above, pasteurisation has been found not to deactivate XOR.

As indicated above, advantageously, the first portion is in a first container, the second portion is in a second container. Thus the two portions can be held separate until use. For example, where both portions are in the form of a powder, each powder portion can be made up separately using different methods before being mixed together. For example, the first portion might be made up with cold water and the second portion may be made up with boiling water. Alternatively, the first portion may be in the form of a liquid ready for mixing into the second portion of the formulation once the second portion has been made up.

Advantageously, the formulation further includes electron donors and/or electron acceptors. Examples of electron donors are purines and nitrogen-containing heterocyclic compounds, for example hypoxanthine, NADH. Examples of electron acceptors are organic and inorganic nitrates and nitrites.

It is envisaged that the composition containing the active XOR might be sold separately from the formula feed, the composition being for addition to the feed prior to use.

Advantageously, the composition comprising active XOR comprises buttermilk. As indicated above, buttermilk is a particularly preferred source of active XOR. Lyophilised buttermilk may be used. It may be preferable, however, for the buttermilk to be spraydried by spraying the buttermilk into air at a temperature which is so selected that the activity of the XOR is wholly or at least substantially retained. In general, to avoid any detrimental effect on the activity of XOR, heat treatment steps that may be used in the treatment of XOR-containing compositions or formulations should preferably be such that the temperature of the composition or formulation does not exceed 65°C.

The composition comprising active XOR may be in the form of a powder.

Advantageously, the composition comprising active XOR further includes electron donors and/or electron acceptors. Examples of electron donors are purines and nitrogen-containing heterocyclic compounds, for example hypoxanthine, NADH. The nature of the electron donor may influence the rate of generation of bactericidal species. For example, use of hypoxanthine as electron donor results in a more rapid, but less prolonged generation of bactericidal species than NADH. Examples of electron acceptors are organic and inorganic nitrates and nitrites.

Preferably, the powder feed formulation comprises a sterilised feed composition.

It will be appreciated that XOR used in accordance with the invention may be of any biological origin, for example of mammalian or other animal origin, or originating from a suitable micro-organism, for example, aspergillus sp. XOR of ruminant origin offers the advantage of ready availability.

The invention will now be explained in more detail with reference to the accompanying drawings, of which:
Fig. 1 is a graph illustrating the effect of peroxynitrite on cell viability, under the conditions of Test 8(a);
Fig. 2 is a graph illustrating the effect of added peroxynitrite on cell colony growth in semi-skimmed-bovine milk, as determined in Test 8(b);
Fig. 3 is a graph illustrating the dependence of XO-mediated killing of cells as determined in Test 8(c);
Fig. 4 is a graph showing the effects of hypoxanthine addition on bacterial growth in pasteurised milk;
Fig. 5 is a graph showing the effect of XO concentration on bacterial growth rate;
Fig. 6 is a graph showing the dependence of cell growth inhibition on hypoxanthine concentration;
Fig. 7 is a graph showing the effect of oxypurinol on XO/hypoxanthine-related growth inhibition; and
Fig. 8 is a graph showing the effect of hypoxanthine at various concentrations on growth inhibition.

### Determination of nitric oxide production

The production of nitric oxide in the following tests and examples was analysed using an ozone chemiluminescence assay in a continuous flow apparatus (Sieves NOA 280) that allows the real time analysis of NO production. The apparatus was modified to allow a constant stream of nitrogen to flow into the reaction chamber. Chemiluminescence data were collected by a data acquisition system; the mean NO produced in parts per billion (ppb) was calculated from readings taken every second and shown as ppb or mV.

Progress curves, of molar production of NO against time, were calculated by taking into account the gas flow and the mean level of NO. Molar production of NO was expressed as ppb/sec or mV/sec. Reactions were carried out in a final volume of 1 ml at 37°C in an atmosphere of < 1% oxygen (Stathkelvin combination needle oxygen electrode, Diamond General Corp.).

The method used was as follows:
(a) Two clean 7ml bijous were obtained, one for each of the "substrates" and the "milk". To the "substrates" bijou, 200µl of 100mM stock sodium nitrite was added together with 200µl of the 5mM reduced NADH to give an assay concentration of 20mM nitrite and 1mM reduced NADH.
   To the "milk" bijou were added 600µl of milk sample to give an assay volume of 1ml.
(b) Using a flow rate of 200ml/min, each bijou mixture and a corresponding injection needle was degassed with nitrogen gas (N₂) for about 10 seconds and the bijou was capped.
(c) The reaction cell comprised a 7ml screw-cap bijou having three needle holes in its cap. A continuous flow of warmed N₂ (200ml/min flow rate) was injected into the bijou through one of the needle holes in the cap to give the required hypoxic conditions. The reaction cell was held at a temperature of 37°C in a water bath mounted on a magnetic stirrer-. A magnetic flea was placed in the reaction cell to mix the samples once injected.
(d) Samples of NO were taken from the reaction cell by a needle that was connected to a Sievers Nitric Oxide Analyser (NOA-280) and the results were recorded for analysis using a computer.
(e) At time (t) 0 minutes, the NOA-280 started the measurement of NO from the reaction cell. At t=1 minute, the contents of the "substrates" bijou were injected into the reaction cell using a 1ml syringe and the background NO was measured.
(f) At t=5 minutes, the contents of the "milk" bijou were injected into the reaction cell using a 1ml syringe and the release of NO was monitored for a further 20 minutes. The set up was such that the reaction cell was a sealed system having an inlet gas flow of 200ml/min and a sample extraction flow to the NOA-280 of 200ml/min. The steady-state generation of NO (which corresponded to a plateau region on the trace of the NO production) was noted to give the mV/s release of NO from the 1ml assay volume.

The samples were diluted with PBS where necessary to give an assay of 1ml for the test of NO generation.

### Reagents used

The reagents used in the tests and examples described below were as follows:
1. Bovine xanthine oxidase (XOR) - Biozyme, Blaenavon, UK. This source of enzyme had a concentration of 10.7mg/ml and was batch 104AX
2. Sodium nitrite (NaNO₂) - Sigma Chemicals (Sigma-Aldrich Company Ltd.), Poole, UK. This was dissolved in 1X Phosphate Buffered Saline (PBS), pH 7.3, to the required concentration.
3. β-Nicotinamide Adenine Dinucleotide, reduced form, (β-NADH) - Sigma Chemicals.
4. Phosphate Buffered Saline (PBS) - Oxoid Ltd., Basingstoke, UK. Tablets were added in the proportion of 1 per 100ml of distilled water and mixed until thoroughly dissolved to give 1X PBS, pH7.3.
5. Oxypurinol - Sigma Chemicals. A stock 1mM solution was made up by adding 0.0015g to 0.25ml of 1M NaOH. This was mixed until the oxypurinol dissolved. Then 9.75ml of 1X PBS was added and the pH altered until pH7.3 was reached using drops of 1M Hl.
6. Diphenyliodonium (DPI) - ICN Biomedical. A stock 1mM solution was made up by adding 0.0032g to 10ml 1X PBS, pH7.3.
7. Formula milks:
   a. PreAptimil with Milupan (2.4g protein/100ml) - Milupa, Trowbridge, UK.
   b. Aptamil First with Milupan (1.5g protein/100m1) - Milupa.
   c. Farleys First Milk - H.J. Heinz Co. Ltd., Hayes, UK.
   d. Cow and Gate Premium (1.4g protein/100ml) - Cow and Gate, Trowbridge, UK.
   e. Sma Gold 1.5g protein/100ml) - SMA Nutrition, Maidenhead, UK.
   f. Sma Wysoy (1.8g protein/100ml) - SMA Nutrition.
8. Human Breast Milk. Samples obtained from subjects in the local area
9. Carton milk. Milk bought in pints from the local shop either as full milk or semi-skimmed milk (3.4g protein/100ml). Lordswood Dairy, Bristol, UK was the source of the milk.
10. Untreated cows' milk was obtained from a local farm
11. Buttermilk obtained from Waitrose (John Lewis Partnership, UK).

### Test 1

The release of nitric oxide (NO) from a composition including pure bovine xanthine oxidase under hypoxic conditions was studied. The samples studied comprised reduced β-Nicotinamide adenine dinucleotide (NADH) and nitrite (NO₂⁻) and pure bovine xanthine oxidase.

Bovine xanthine oxidase was diluted with PBS as indicated in Table 1 below to give 300µl of enzyme mixture. The enzyme mixture (300µl) was mixed with 700µl of a substrate mixture to give an assay volume of 1 ml. The substrate mixture comprised nitrite which was added to the assay to give a concentration of 1mM and NADH which was added to give a concentration of 0.3mM. Where, for example in Tests 2 to 4 below, additional components are added to the sample, the amount of PBS is adjusted accordingly to give an assay volume of 1 ml. The total XOR protein in the assay is shown in Table 1. The sample was placed in the reaction vessel of the NO determination apparatus under a nitrogen atmosphere and the generation of NO was measured and the results calculated as a steady state rate in mV/s. The rate of NO release for the different concentrations of XOR is also shown in Table 1.

**Table 1**

| **Volume XOR enzyme (µl)** | **Volume PBS (µl)** | **XOR protein in assay (µg)** | **NO release (mV/s)** |
|---|---|---|---|
| 0 | 300 | 0.0 | 15.10 |
| 2 | 298 | 21.4 | 50.05 |
| 5 | 295 | 53.5 | 76.35 |
| 10 | 290 | 107.0 | 128.55 |
| 15 | 285 | 160.5 | 187.20 |
| 20 | 280 | 214.0 | 225.75 |

Where the pure XOR is used in the samples, NADH was required as a substrate for the reduction of the nitrite to proceed. It is believed that in many cases, where the XOR is added to, for example a formula feed, the addition of a separate electron donor, for example NADH, will not be required because suitable substrate species will already be present in the feed and/or in the GI tract of the infant or adult to whom the feed is administered.

### Test 2

Using the method described above in respect of Test 1, the effect of the variation in the concentration of NADH on the production of NO was investigated. 4mM NADH was diluted with PBS to give the relevant concentration. The results are shown in Table 2.

**Table 2**

| **XOR protein in assay (µg)** | **NADH concentration in assay (mM)** | **NO release (mV/s)** |
|---|---|---|
| 53.5 | 0.00 | 0.00 |
| 53.5 | 0.10 | 30.45 |
| 53.5 | 0.25 | 66.80 |
| 53.5 | 0.50 | 92.55 |
| 53.5 | 1.00 | 103.35 |
| 53.5 | 2.00 | 109.20 |
| 107.0 | 0.00 | 0.00 |
| 107.0 | 0.10 | 74.45 |
| 107.0 | 0.25 | 142.70 |
| 107.0 | 0.50 | 192.65 |
| 107.0 | 1.00 | 230.05 |
| 107.0 | 2.00 | 264.65 |

### Test 3

Using the method described above in respect of Test 1, the effect of the variation in the concentration of nitrite on the production of NO was investigated. As for Test 1, the concentration of NADH was 0.3 mM. 1M nitrite (or 100mM nitrite, where appropriate for low concentrations) was diluted with PBS to give the relevant concentration. The results are shown in Table 3.

**Table 3**

| **XOR protein in assay (µg)** | **Nitrite concentration in assay (mM)** | **NO release (mV/s)** |
|---|---|---|
| 21.4 | 0 | 0.00 |
| 21.4 | 1 | 53.75 |
| 21.4 | 5 | 184.8 |
| 21.4 | 10 | 378.10 |
| 21.4 | 25 | 407.55 |
| 21.4 | 50 | 474.05 |

### Test 4

The method of Test 1 was repeated with known inhibitors of XOR included in the assay to show that the NO generation was being catalysed by XOR. Two different inhibitors were used. Oxypurinol was used at a concentration of 100µM. Oxypurinol is a molybdenum site-specific inhibitor. Diphenyliodonium (DPI) was used at a concentration of 100 µM. DPI is a FAD site inhibitor. The results are shown in Table 4.

**Table 4**

| **Inhibitor** | **NO release (mV/s)** |
|---|---|
| 100 µM Oxypurinol | 0.00 |
| 100 µM DPI | 0.00 |

There was no release of NO in the presence of the XOR inhibitors.

### Test 5

Full fat milk was assayed with 20mM nitrite and 1mM NADH as described above in Test 1. The milk had a protein content of 3.4g/100ml. 600µl of milk was used in each test giving a protein content in each assay of 20.4mg.

The milk was taken and divided into two halves. One was kept at 4°C, and the other at -20°C. Samples of the milk were taken over several days and the generation of NO was investigated using the method described above. The results are given below in Table 5.

**Table 5**

| **Day** | **4°C - NO release (mV/s)** | **-20°C - NO release (mV/s)** |
|---|---|---|
| 1 | 77.57 | 73.00 |
| 2 | 75.50 | 90.05 |
| 3 | 68.67 | 52.65 |
| 6 | 626.1 | 73.35 |
| 8 | Over 1000 | 39.20 |

It was found that the milk which was kept at 4°C started to go off after 3 days and that that was accompanied by a large increase in NO release which was not inhibited by 100µM oxypurinol or DPI. Therefore, when the milk goes off it is probable that bacteria in the milk are starting to produce NO from the nitrite. The milk kept frozen retained its activity-and did not go off.

Samples of fresh full fat milk assay with 100µM Oxypurinol or DPI were tested and found to give no detectable NO generation. Thus full fat milk does include a source of active XOR which produces NO under hypoxic conditions with nitrite and NADH.

### Test 6

Human breast milk was assayed with 20mM nitrite and 1mM NADH using the method as described above. The human breast milk was collected on several days post partum and frozen at -20°C. 600µl of milk was used in each test. The generation of NO was investigated using the method described above. The results are given below in Table 6.

### Table 6

| **Days post partum** | **XOR protein content (µg/ml)** | **NO release (mV/s)** |
|---|---|---|
| 7 | 411.26 | 40.45 |
| 30 | 683.05 | 60.75 |
| 36 | 561.49 | 42.48 |
| 66 | 305.52 | 27.80 |
| 158 | 110.84 | 29.80 |

### Test 7

A selection of formula feeds (formula milks) were taken and assayed with 20mM nitrite and 1mM NADH as described above. The formula milks were tested for the generation of NO. The Cow and Gate Premium was left for 7 days after opening and then tested again. The results are shown in Table 7.

**Table 7**

| **Formula feed** | **NO release (mV/s)** |
|---|---|
| Cow and Gate Premium (fresh) | 0.00 |
| Cow and Gate Premium (7 days old) | Over 1000 |
| Milupa Aptimil First with Milupan | 0.00 |
| Sma Wysoy | 0.00 |
| Sma Gold | 0.00 |
| Farleys First | 0.00 |
| Milupa Preaptimil with Milupan | 0.00 |

There was no activity found in any of the fresh formula milks. The Cow and Gate formula had gone off after 7 days and the NO release is probably related to bacteria (the activity was not inhibited by 100µM oxypurinol or DPI).

### Test 8

To obtain cells used in this test, the infective bacterial strain Escherichia coli NCTC 86 (E.coli) was cultured on nutrient agar at 37° until colony formation occurred, usually overnight. This stock culture was used for subsequent experiments including sub-culturing in nutrient broth and plated onto agar weekly. Experimental cultures of E.coli were set up overnight in nutrient broth from single colonies on an agar plate. Cells were harvested and counted using a standard curve of known absorbance at 470nm against viable cell count. Peroxynitrite was generated by the method of Crow et al, Biochem. 34, p.3544-3552 (1995). In accordance with that method, a mixture of sodium nitrite and hydrogen peroxide was reacted under acid conditions then immediately quenched by the addition of sodium hydroxide. The concentration of ONOO⁻ formed was measured using an absorption coefficient of 1670 M⁻¹ cm⁻¹ in a spectrophotometer at a wavelength of 303nm. Solutions of different concentrations for use in (a) below were obtained by dilution of the product solution using PBS.

### 8(a) Peroxynitrite effect on cell viability

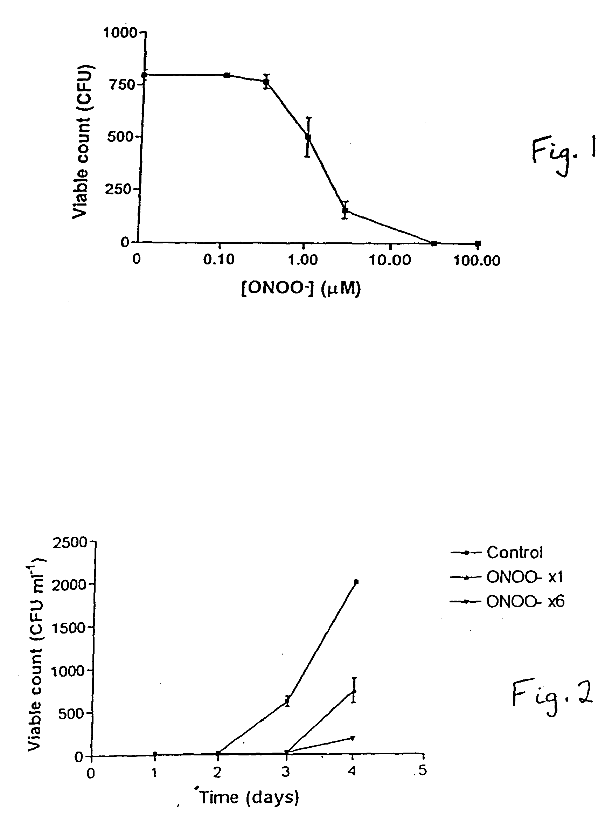

Cells cultured as described above were diluted to suitable working concentrations (10⁴ - 10⁵ Cells Ml⁻¹) in sterile phosphate buffered saline (PBS). ONOO⁻ at a range of concentrations from 100µm to 0.01µm was added as a bolus dose to cells and incubated at room temperature for ten minutes. Aliquots were taken from the cell cultures and plated on to nutrient agar and incubated in a warm room at 37°C overnight. Viable cells formed colonies on the agar and were counted. The number of colonies formed was related to an untreated control and a graph of the results is shown in Figure 1, which illustrates the effect of ONOO⁻ on cell viability. The decrease in viable count with increasing concentration of ONOO⁻ in Fig. 1 is indicative of a profound inhibitory effect. Peroxynitrite has also been shown to reduce viability in S. enteritidis under similar conditions to those indicated above. IC₅₀ values of peroxynitrite in respect of E. coli and S. enteritidis (that is, the concentration that reduces viability to 50% of control viable count of the respective cell type) of 1.402 and 2.026µM were determined under the conditions used. Peroxynitrite-medicated killing has also been indicated in the case of Gram positive bacteria, in a test in which incubation of Staphylococcus aureus with SIN-1 (3-morpholinosydnonimine, which releases both superoxide and NO simultaneously on hydration) showed reduced growth in dependence on the amount of added SIN-1.

### 8(b) Effect of peroxynitrite addition to bovine milk

Commercially produced bovine milk was purchased in semi-skimmed form. Aliquots were taken on day one of experimentation and plated onto nutrient agar and grown overnight at 37°C. The number of colonies formed following incubation was counted. The remaining milk was divided into three portions after removal of those aliquots. No peroxynitrite was added to the first portion, which was the control. A single bolus dose of 100µM ONOO⁻ was added to the second portion on day one, and the third portion was treated by addition of a daily bolus dose of 100µM ONOO⁻. The portions were stored during the four days of the test at a temperature of 4°C. Each day, aliquots were taken from each portion and cultured overnight at 37°C for viable count determination.

The results are shown in Fig. 2 and demonstrate that ONOO⁻ addition to milk caused a reduction in the number of colony forming units (CFU) over time compared to the control. The single bolus addition reduced the CFU significantly (p<=0.01) and the multiple dose ONOO⁻ reduced the CFU significantly below control (p<=0.01) and also significantly below the single dose ONOO⁻ (p<=0.05). Although the contaminants were not formally identified, it was postulated that they included at least Lactobacilli sp., that being a cell type often present in milk.

### 8(c) Effect of xanthine oxidase derived species on cell viability

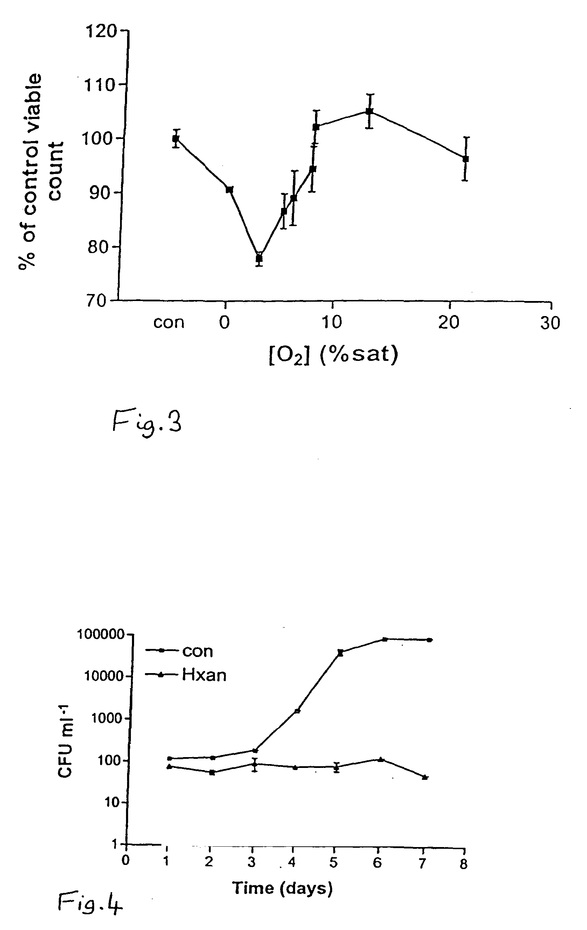

E.coli were cultured as described above. Aliquots were taken and incubated with a reaction system consisting of bovine Xanthine oxidase (XO) (53.2 µgml⁻¹), nicotinamide adenine dinucleotide in reduced form (NADH) (300µM), sodium nitrite, (NaNO₂) 1mM and oxygen at a range of concentrations. Desired oxygen concentrations were generated by delivery into the system of a mixture of oxygen and nitrogen in appropriate proportions and determined using a Clark-type 0₂ electrode. This reaction was followed at 37°C for 30 min with mixing before an aliquot was taken and plated onto agar and incubated in a warm room at 37°C. Viable cells formed colonies on the agar and were counted. Viable cell counts were performed in triplicate and the results expressed as a percentage viable count in each case related to a non enzyme control of the same oxygen concentration. The results are shown in Fig. 3, which suggest that XO-mediated killing of cells is occurring. The most effective killing (indicated by the lowest viable cell count) is at an oxygen concentration of approximately 3% of saturation. The range of oxygen tensions used covers those in which XO has previously been considered to be active, namely superoxide generation (21% O₂ saturation) and nitric oxide production (0% O₂ saturation). A certain amount of killing is seen at both of these extremes as compared with control samples. However, it is only at an intermediate oxygen concentration that the greatest amount of killing is observed. This suggests a role for peroxynitrite mediated killing which has been generated in this system by the enzyme xanthine oxidase. Corresponding data obtained in respect of S. enteritidis indicated a peak killing oxygen concentration of 0% for that cell type. For both E. coli and S. enteritidis the viability increases with oxygen concentration with little or no killing above about 8% oxygen, although some limited killing (about 5% and about 10% for the respective cell types) is again observed at higher oxygen concentrations of about 21%.

Replacement of NADH in the above method by 100µM hypoxanthine, with a sodium nitrite concentration of 2.5µM led to slightly higher peak killing oxygen concentrations (6.4% for E. coli and 1.5% for S. enteritidis). More limited killing was also observed using xanthine instead of hypoxanthine. The XO/hypoxanthine combination was also found to reduce the growth rate of Lactobacillus in a dose dependent manner for both hypoxanthine and XO. Addition to the XO/hypoxanthine system of superoxide dismutase at oxygen concentrations in the range of from 0 to 2% was found to increase the amount of measurable NO (as a result of removal of superoxide by superoxide dismutase), providing a further indication of the hypothesis that peroxynitrite formation from NO and superoxide occurs in the XO/hypoxanthine system.

### 8(d) Effect of peroxynitrite scavenger on bacterial growth

E. coli were seeded into nutrient broth at 2.10⁷ cell ml⁻¹ and incubated at 37°C under atmospheric air conditions (that is, 21% oxygen saturation). Four separate test samples of volume 1 ml were prepared by addition at 1 hour of incubation as follows:
1 Peroxynitrite at concentration 100µM.
2 Peroxynitrite at 100µM and quercetin (peroxynitrite scavenger) at 100µM.
3 Xanthine oxidase (53.2µg) and xanthine at 100µM.
4 Xanthine oxidase (53.2µg) and NADH at 100µM.

Corresponding controls were also prepared, and the growth curves generated over time determined by monitoring absorbance. Sample (1) showed strong killing, but the presence of quercetin (sample (2)) had a clear effect in reducing killing, pointing towards peroxynitrite mediation of killing. XO/NADH (sample (4)) and XO/xanthine (sample (3)) showed limited, but significant, growth retardation effects.
The results of parts (a), (b) and (d) of this test appear to confirm the bactericidal potency of the peroxynitrite species. Part (c) above supports the hypothesis that, under appropriate conditions, XOR can catalyse production both of superoxide and of NO, interaction of those two products giving rise to peroxynitrite, and possibly other interaction products that may have similar bactericidal activity to peroxynitrite. The oxygen concentrations of under 8% at which maximum killing was observed is believed to be similar to that in the neonatal gut.

### 8(e) Effect of hypoxanthine addition on bacterial growth in pateurised milk.

Pasteurised semi skimmed milk 1 ml was aliquoted into 7 sterile plastic bijou bottles comprising control and treated hypoxanthine (Hxan) groups. To the control group at day 0 was added 10µl of sterile ddH₂0 and to the treated group 10µl of hypoxanthine solution was added to give a final concentration of 100µM. The samples were stored refrigerated at 4-8°C until required on a specific day when a control and treated sample was incubated at 37°C for 30 minutes. A 100µl aliquot was taken from each sample and spread onto nutrient agar which was then incubated over night at 37°C. The total number of colonies formed regardless of type was counted and expressed for each treatment as the colony forming units per ml of the original test sample (CFU ml⁻¹). The results are shown in Fig. 4.
The control group showed significant colony formation from day 1. The effect of the addition of hypoxanthine was to reduce the total number of colonies formed for the length of the experiment (8 days).

### 8(f) Effect of XO and hypoxanthine incubation on bacterial growth rate.

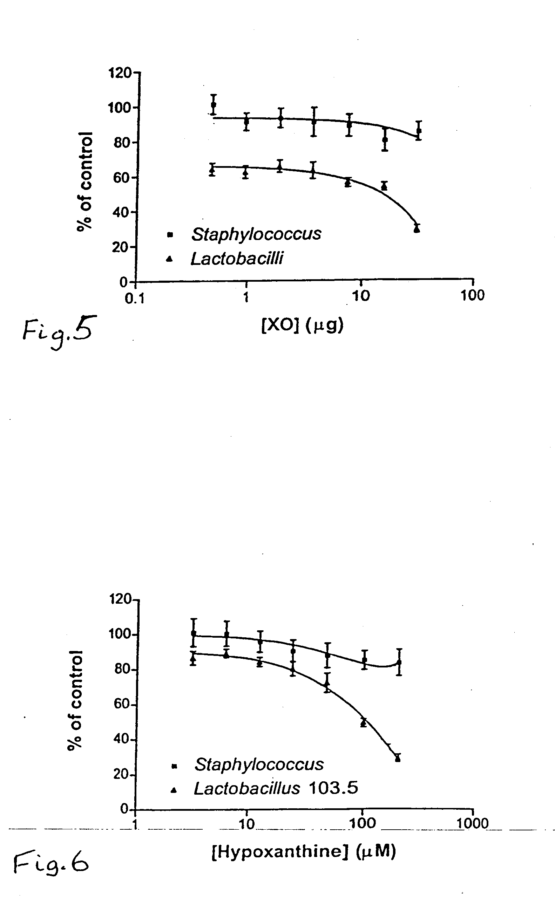

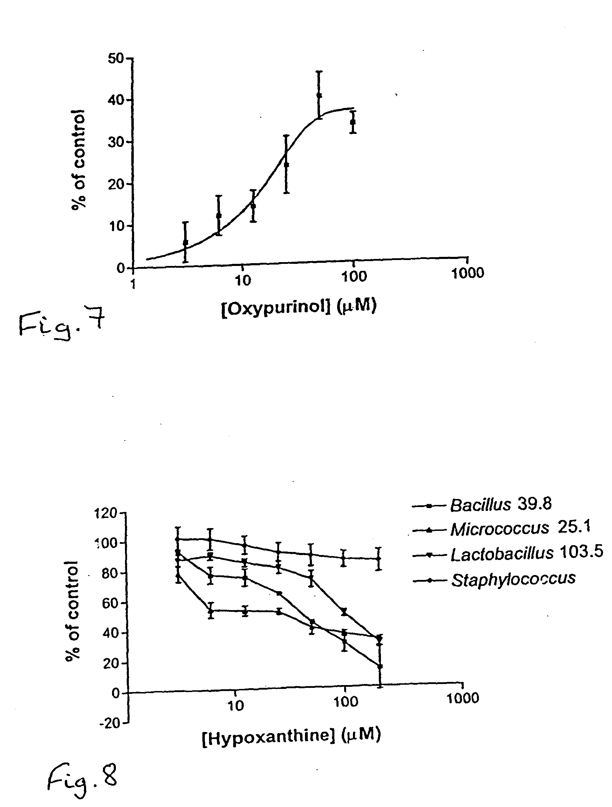

Bacteria were seeded into nutrient broth (NB) and grown over night at 37°C. The culture was then counted and normalised to give a final cell concentration of 1.8x10⁷ cells well⁻¹ into the wells of a 96 well plate in fresh nutrient broth. The optical density of each well was monitored every 15 minutes as a measure of the growth rate of each bacterial species. Growth curves were generated for each species and the maximal rate of growth (logarithmic phase) was measured. Cells were treated to a range of experimental conditions in which the growth rate of cells was related to a control of untreated cells. The results were expressed as a percentage of the growth rate of the control cells.
(i) At a fixed concentration of hypoxanthine (100µM) a range of purified XO protein concentrations were added at time 0 minutes. The optical density was followed and the growth rate calculated. The effect of enzyme concentration is shown in Fig. 5 for *Staphylococcus aureus* 6751 and *Lactobacillus casei* 6375.
   The growth rate of both Staphylococcus and Lactobacilli was reduced compared to the control. Lactobacillus growth rate was reduced with a half maximal concentration of XO (that is, the concentration at which the cell growth was 50% of control) being about 14µg. A less marked growth inhibition was observed for Staphylococcus.
(ii) The growth rate was also measured in relation to the concentration of hypoxanthine, using 30µg of XO with cells seeded as described above. Fig. 6 shows the effect of hypoxanthine concentration on bacterial growth. Hypoxanthine in the presence of XO reduced the growth rate of both bacterial species with greatest effect on the Lactobacillus. The half maximal hypoxanthine dose at 30µg XO was 103.5µM.
(iii) To show that the effect of XO and Hypoxanthine addition was due to the enzymically derived products, oxypurinol at a range of concentrations was added to the cells in the presence of 30µg XO and 200µM hypoxanthine. Fig. 7 shows its effect on the growth rate of Lactobacillus.
   Oxypurinol had no effect on growth rate when added at the highest concentration when added alone.
   However the effect of oxypurinol was to reduce the effect of XO/hypoxanthine growth inhibition in a dose dependent manner.
(iv) The effect of XO hypoxanthine addition was measured on the growth rate of a range of bacterial species with 30µg XO at varying hypoxanthine concentrations using cells seeded as described above. The results of this study are shown in Fig. 8.

### 8(g) Effect of H₂O₂ and peroxynitrite on the growth rate of bacteria

To determine the effect of possible enzymically generated radical species cells were grown in the presence of hydrogen peroxide or peroxynitrite. Cells of various species were grown as previously described and a bolus addition of either H₂O₂ or peroxynitrite was added at the beginning of logarithmic growth, previous experiments having showed this treatment as the most effective. The cell growth was determined and plotted against the concentration of H₂O₂ or peroxynitrite, as appropriate, and the half maximal concentration of H₂O₂ or peroxynitrite was determined. The results are summarised in Table 8, in which corresponding half maximal concentrations for the XO/hypoxanthine system are also given.

**Table 8**

| | **Half maximal concentration** | | |
|---|---|---|---|
| Species | XO/Hxan (µM Hypoxanthine) | H₂O₂ (µM) | ONOO - (µM) |
| *Bacillus sp* | 39.8 | 77.6 | |
| *Micrococcus sp* | 25.1 | 19.5 | |
| *Lactobacillus casei* | 103.5 | 304.8 | 55 |
| *Staphylococcus aureus* | | | 290 |
| *E. coli* | | | 1.4 |
| *Salmonella enteritidis* | | | 2.0 |

As shown in Table 8, the half maximal concentrations for peroxynitrite in respect of Lactobacillus casei is much lower than for H₂O₂. In respect of S. aureus, the growth inhibition in the presence of H₂O₂ was very limited, with 50% growth reduction not being observed at the concentration ranges used.

The following Example illustrates the invention:

### Example 1

The effect of the addition of XOR to formula feed on the generation of NO was investigated. Different compositions comprising one or more of buttermilk, formula feed, XOR, nitrite and NADH were studied under hypoxic conditions.

The samples were tested for NO generation using the method described above.

To the "milk" bijou were added either 598µl Cow and Gate formula feed and 2µl XOR or 100µl buttermilk and 500µl 1X PBS to give a total volume in the "milk" bijou of 600µl in each case.

**Table 9**

| **Sample** | **20mM nitrite added?** | **1mM NADH added?** | **NO release (mV/s)** |
|---|---|---|---|
| Formula feed and XOR | Yes | Yes | 247.3 |
| Formula feed and XOR | No | No | 0.0 |
| Formula feed and XOR | Yes | No | 0.0 |
| Formula feed and XOR | No | Yes | 0.0 |
| Formula feed and 1X PBS | Yes | Yes | 0.0 |
| Buttermilk | Yes | Yes | 379.1 |

Table 9 shows that where no XOR is added to the formula feed, there is no generation of NO. That is consistent with the applicant's findings that formula feeds do not contain active XOR.

The addition of XOR to the formula feed in the presence of nitrite and NADH led to the generation of NO. The omission of either the nitrite or the NADH gave no NO generation. As indicated above, it is believed that both a source of nitrite and an electron donor substrate will be available *in vivo* in the GI tract and that in many cases specific further addition of nitrite and/or NADH (or other substrate) will not be required.

Buttermilk alone, without the addition of any of XOR, nitrite or NADH, led to the generation of NO. As indicated above, buttermilk contains active XOR as well as a source of nitrite, and an electron donor substrate.

## Claims

1. A method of making a formula feed for a human baby, the method comprising manufacturing a powder feed formulation, the powder feed formulation so obtained being substantially free of active xanthine oxidoreductase (XOR), and further comprising adding to that powder feed formulation a composition comprising active XOR.

2. A method according to claim 1, in which the amount of the composition comprising active XOR added is such that the formula feed, when reconstituted, includes from 50µg/ml to 500µg/ml active XOR.

3. A method according to claim 1 or claim 2, in which the amount of the composition comprising active XOR added is such that the concentration of XOR in the formula feed, when reconstituted, exceeds the normal physiological concentration of XOR.

4. A method according to claim 2 or claim 3, in which the amount of the composition comprising XOR added is such that the formula feed, when reconstituted, includes from 50 to 150µg/ml of XOR.

5. A method according to any one of claims 1 to 4, in which the formula feed further includes one or more electron donors.

6. A method according to any one of claims 1 to 5, in which the formula feed further includes one or more electron acceptors.

7. A method according to any one of claims 1 to 6, further comprising a step of adding water to obtain a liquid feed for administration to said baby.

8. A method according to any one of claims 1 to 7, in which said composition comprising active XOR includes XOR in combination with one or more electron donors and/or one or more electron acceptors.

9. A method according to any one of claims 1 to 8, in which said composition comprising XOR is in the form of a powder.

10. A method according to any one of claims 1 to 9, in which said composition comprising active XOR has been obtained by spray-drying buttermilk at a temperature which is so selected that the activity of the XOR is at least substantially retained.

11. A method according to any one of claims 1 to 10, in which said composition comprising active XOR has been subjected to a heat treatment step in which its temperature does not exceed 65°C.

12. A method according to any one of claims 1 to 11, in which the powder feed formulation is sterilised.

## Patentansprüche

1. Verfahren zur Herstellung einer formelmäßigen Nahrung für ein menschliches Baby, wobei das Verfahren die Herstellung einer pulverförmigen Nahrungsmittel-Formulierung umfasst, und die so erhaltene pulverförmige Nahrungsmittel-Formulierung im Wesentlichen frei von aktiver Xanthin-Oxidoreduktase (XOR) ist, und wobei das Verfahren darüber hinaus die Zugabe einer Zusammensetzung, die aktive XOR umfasst, zu dieser pulverförmigen Nahrungsmittel-Formulierung umfasst.

2. Verfahren nach Anspruch 1, bei welchem die zugegebene Menge der Zusammensetzung, die aktive XOR umfasst, dergestalt ist, dass die formelmäßige Nahrung, wenn sie wiederhergestellt wird, 50 µg/ml bis 500 µg/ml aktive XOR enthält.

3. Verfahren nach Anspruch 1 oder Anspruch 2, bei welchem die zugegebene Menge der Zusammensetzung, die aktive XOR umfasst, dergestalt ist, dass die Konzentration der XOR in der formelmäßigen Nahrung, wenn sie wiederhergestellt wird, die normale physiologische Konzentration der XOR übersteigt.

4. Verfahren nach Anspruch 2 oder Anspruch 3, bei welchem die zugegebene Menge der Zusammensetzung, die XOR umfasst, dergestalt ist, dass die formelmäßige Nahrung, wenn sie wiederhergestellt wird, 50 µg/ml bis 150 µg/ml XOR enthält.

5. Verfahren nach einem der Ansprüche 1 bis 4, bei welchem die formelmäßige Nahrung darüber hinaus einen oder mehrere Elektronen-Donoren enthält.

6. Verfahren nach einem der Ansprüche 1 bis 5, bei welchem die formelmäßige Nahrung darüber hinaus einen oder mehrere Elektronen-Akzeptoren enthält.

7. Verfahren nach einem der Ansprüche 1 bis 6, welches darüber hinaus einen Schritt der Zugabe von Wasser umfasst, um eine flüssige Nahrung für die Verabreichung an das Baby zu erhalten.

8. Verfahren nach einem der Ansprüche 1 bis 7, bei welchem die Zusammensetzung, die aktive XOR umfasst, XOR in Kombination mit einem oder mehreren Elektronen-Donoren und / oder mit einem oder mehreren Elektronen-Akzeptoren enthält.

9. Verfahren nach einem der Ansprüche 1 bis 8, bei welchem die Zusammensetzung, die XOR umfasst, in Form eines Pulvers vorliegt.

10. Verfahren nach einem der Ansprüche 1 bis 9, bei welchem die Zusammensetzung, die aktive XOR umfasst, durch Sprühtrocknen von Buttermilch bei einer Temperatur erhalten wurde, welche so ausgewählt wurde, dass die Aktivität der XOR zumindest im Wesentlichen erhalten bleibt.

11. Verfahren nach einem der Ansprüche 1 bis 10, bei welchem die Zusammensetzung, die aktive XOR umfasst, einem Schritt der Wärmebehandlung unterzogen wurde, bei dem die Temperatur 65 °C nicht übersteigt.

12. Verfahren nach einem der Ansprüche 1 bis 11, bei welchem die pulverförmige Nahrungsmittel-Formulierung sterilisiert wird.

## Revendications

1. Procédé de préparation d'un aliment composé pour bébé humain, le procédé comprenant la fabrication d'une composition d'aliment en poudre, la composition d'aliment en poudre ainsi obtenue étant pratiquement exempte de xanthine oxydoréductase (XOR) active, et en outre comprenant l'addition à cette composition d'aliment en poudre d'une composition comprenant de la XOR active.

2. Procédé selon la revendication 1, dans lequel la quantité de la composition comprenant de la XOR active ajoutée est telle que l'aliment composé, lorsqu'il est reconstitué, inclut de 50 µg/ml à 500 µg/ml de XOR active.

3. Procédé selon la revendication 1 ou 2, dans lequel la quantité de la composition comprenant de la XOR active ajoutée est telle que la concentration de XOR dans l'aliment composé, lorsqu'il est reconstitué, excède la concentration physiologique normale de la XOR.

4. Procédé selon la revendication 2 ou 3, dans lequel la quantité de la composition comprenant de la XOR ajoutée est telle que l'aliment composé, lorsqu'il est reconstitué, inclut de 50 à 150 µg/ml de XOR.

5. Procédé selon l'une quelconque des revendications 1 à 4, dans lequel l'aliment composé en outre inclut un ou plusieurs donneurs d'électrons.

6. Procédé selon l'une quelconque des revendications 1 à 5, dans lequel l'aliment composé en outre inclut un ou plusieurs accepteurs d'électrons.

7. Procédé selon l'une quelconque des revendications 1 à 6, comprenant en outre une étape d'addition d'eau pour l'obtention d'un aliment liquide pour administration audit bébé.

8. Procédé selon l'une quelconque des revendications 1 à 7, dans lequel ladite composition comprenant de la XOR active inclut de la XOR en association avec un ou plusieurs donneurs d'électrons et/ou un ou plusieurs accepteurs d'électrons.

9. Procédé selon l'une quelconque des revendications 1 à 8, dans lequel ladite composition comprenant de la XOR est sous la forme d'une poudre.

10. Procédé selon l'une quelconque des revendications 1 à 9, dans lequel ladite composition comprenant de la XOR active a été obtenue par séchage de babeurre par atomisation à une température qui est choisie de manière que l'activité de la XOR soit au moins pratiquement conservée.

11. Procédé selon l'une quelconque des revendications 1 à 10, dans lequel ladite composition comprenant de la XOR active a été soumise à une étape de traitement thermique dans laquelle sa température n'excède pas 65 °C.

12. Procédé selon l'une quelconque des revendications 1 à 11, dans lequel la composition d'aliment composé est stérilisée.
